# EUROPEAN PATENT APPLICATION

(11) **EP 4 397 739 A1**
(43) Date of publication of application: **10.07.2024**
(21) Application number: 22863509.0
(22) Date of filing: 31.08.2022
(51) Int. Cl.: C11D 3/386, A62D 3/02

(54) **DECONTAMINATION COMPOSITION AND APPLICATION THEREOF**

(30) Priority: 03.09.2021 CN 202111032492
(71) Applicant: Academy of Military Medical Sciences, Beijing 100850 (CN)
(72) Inventor: ZHONG, Wu, Beijing 100850 (CN); HE, Qinghao, Beijing 100850 (CN); LI, Song, Beijing 100850 (CN)
(74) Representative: PGA S.p.A., Milano, Succursale di Lugano
(86) International application number: PCT/CN2022/116137
(87) International publication number: WO 2023/030374

(57) **Abstract**

A decontamination composition, containing chloroperoxidase. The decontamination composition can rapidly degrade chemical warfare agents, namely mustard gas (HD), Lewisite (L) and VX, and the decontamination products of mustard gas and Lewisite are non-toxic, thereby effectively preventing the harm of the chemical warfare agents to the environment and organism, and reducing the degree of harm of the products to soil, vegetation and organism compared with conventional decontaminant agents.

## Description

The present application is based on the application with the CN application number of 202111032492.9 and the filing date of September 3, 2021, and claims its priority. The disclosure of the CN application is hereby incorporated into this application in its entirety.

### Technical Field

The present application relates to the fields of environmental decontamination and biological organism decontamination, and specifically relates to a composition particularly suitable for decontamination of chemical poisons and application thereof.

### Background Art

Chemical poisons, generally referred to as Chemical Warfare Agents (CWAs), refer to various chemical substances that are used for war purposes, are highly toxic, and can poison or kill enemy humans, animals, and plants on a large scale. There are mainly nerve agents, blister agents, systemic poisons, incapacitating agents, irritant agents, asphyxiating agents, etc.

In order to reduce the harm of chemical poisons, decontamination agents must be used to eliminate their toxic effects, which makes decontamination agents a research hotspot in the field of chemical poison protection.

Commonly used decontamination agents include sodium hypochlorite, DS2 (Decontamination solution 2) and reactive skin decontamination lotion (RSDL). However, these decontamination agents have many shortcomings. For example, sodium hypochlorite and DS2 are corrosive, and are not suitable for general routine applications, and RSDL has adverse effects on skin trauma. Therefore, biofriendly purification methods are still attracting much attention.

### Contents of the present invention

The present application aims to develop a new generation of efficient and safe protease decontamination system to deal with the threats of chemical warfare and chemical terrorism. The efficiency and safety of this system have been verified by in vitro data and animal models, and it can achieve rapid broad-spectrum decontamination within 1 minute, laying a foundation for the development of a new generation of human-friendly decontaminants. Research on the new broad-spectrum enzymatic decontaminant will promote the improvement of military equipment defense system capabilities, the improvement of anti-terrorism systems, and the establishment of national chemical safety systems.

This study overcomes the shortcomings of common methods for degrading chemical poisons (CWAs) and explores chloroperoxidase (CPO)-catalyzed oxidative degradation of chemical poisons, demonstrating that it is a mild and more effective alternative. Under optimized conditions such as enzymes, H₂O₂, Cl⁻, pH, and cosolvent, CPO can effectively degrade sulfur mustard gas (HD), Lewisite (L), Agent Yellow (HD + L), and other types of chemical poisons including VX, and does not generate harmful products.

In one aspect, the present invention provides a decontamination composition, which comprises a chloroperoxidase.

In some embodiments, the chloroperoxidase is CPO (EC 1.11.1.10).

In some embodiments, the decontamination composition further comprises one or more selected from the group consisting of hydrogen peroxide, halide ion, buffer solution, and cosolvent.

In some embodiments, the decontamination composition further comprises an ingredient or combination selected from the group consisting of: hydrogen peroxide; halide ion; buffer solution; cosolvent; hydrogen peroxide and halide ion; hydrogen peroxide and buffer solution; hydrogen peroxide and cosolvent; halide ion and buffer solution; halide ion and cosolvent; buffer solution and cosolvent; hydrogen peroxide, halide ion and buffer solution; hydrogen peroxide, halide ion and cosolvent; hydrogen peroxide, buffer solution and cosolvent; halide ion, buffer solution and cosolvent; and hydrogen peroxide, halide ion, buffer solution and cosolvent.

In some embodiments, the decontamination composition comprises a chloroperoxidase, a hydrogen peroxide, a halide ion, a buffer solution and a cosolvent.

In some embodiments, the decontamination composition is characterized by one or more of the following:
(1) the halide ion is selected from the group consisting of fluoride ion, chloride ion and bromide ion; preferably chloride ion;
(2) the buffer solution is selected from the group consisting of phosphate buffer solution, borate buffer solution, and citrate buffer solution; preferably phosphate buffer solution, such as KH₂PO₄ buffer solution;
(3) the cosolvent is selected from alcohols, such as tert-butyl alcohol.

In some embodiments, the decontamination composition is characterized by one or more of the following:
(1) the chloroperoxidase has a concentration of nanomolar level to millimolar level; for example, 1nM to 100mM, preferably 20nM to 10mM;
(2) the hydrogen peroxide has a concentration of 0 to 50 mM;
(3) the halide ion has a concentration of 0 to 0.5 M;
(4) the buffer solution has a pH of 2.0 to 5.0;
(5) the cosolvent has a concentration of 1 to 10 % (v/v).

In some embodiments, the chloroperoxidase has a concentration of 1nM to 50mM, 1nM to 20mM, 1nM to 10mM, 1nM to 1mM, 1nM to 500nM, 1nM to 200nM, 1nM to 100nM, 1nM to 50nM, 1nM to 30nM, 1nM to 20nM, 1nM to 10nM, 10nM to 100mM, 10nM to 50mM, 10nM to 20mM, 10nMto 10mM, 10nMto 1mM, 10nMto 500nM, 10nM to 200nM, 10nMto 100nM, 10nM to 50nM, 10nM to 30nM, 10nM to 20nM, 20nM to 100mM, 20nM to 50mM, 20nM to 20mM, 20nM to 10mM, 20nM to 1mM, 20nM to 500nM, 20nM to 200nM, 20nM to 100nM, 20nM to 50nM, 20nM to 30nM, 30nM to 100 mM, 30nM to 50mM, 30nM to 20mM, 30nM to 10mM, 30nM to 1mM, 30nM to 500nM, 30nM to 200nM, 30nM to 100nM, 30nM to 50nM, 50nM to 100mM, 50nM to 50mM, 50nM to 20mM, 50nM to 10mM, 50n M to 1mM, 50nM to 500nM, 50nM to 200nM, 50nMto 100nM, 100nM to 100mM, 100nM to 50mM, 100nM to 20mM, 100nM to 10mM, 100nM to 1mM, 100nM to 500nM, 100nM to 200nM, 200nM to 10 0mM, 200nM to 50mM, 200nM to 20mM, 200nM to 10mM, 200nM to 1mM, 200nM to 500nM, 500nM to 100mM, 500nM to 50mM, 500nM to 20mM, 500nM to 10mM, 500nM to 1mM, 1mM to 100mM, 1mM to 50mM, 1mM to 20mM, 1mM to 10mM, 10mM to 100mM, 10mM to 50mM, 10mM to 20mM, 20mM to 100mM, 20mM to 50mM, or 50mM to 100mM.

In some embodiments, the hydrogen peroxide has a concentration of 50nM to 50mM, 50nM to 20mM, 50nM to 10mM, 50nM to 5mM, 50nM to 1mM, 50nM to 500nM, 50nM to 200nM, 50nM to 100nM, 100nM to 50mM, 100nM to 20mM, 100nM to 10mM, 100nM to 5mM, 100nM to 1mM, 100nM to 500nM, 100nM to 200nM, 200nM to 50mM, 200nM to 20mM, 200nM to 10mM, 200nM to 5mM, 200nM to 1mM, 200nM to 500nM, 500nM to 50mM, 500nM to 20mM, 500nM to 10mM, 500nM to 5mM, 500nM to 1mM, 1mM to 50mM, 1mM to 20mM, 1mM to 10mM, 1mM to 5mM, 5mM to 50mM, 5mM to 20mM, 5mM to 10mM, 10mM to 50mM, 10mM to 20mM, or 20mM to 50mM.

In some embodiments, the halide ion has a concentration of 50nM to 0.5M, 100nM to 0.5M, 200nM to 0.5M, 500nM to 0.5M, 1mM to 0.5M, 10mM to 0.5M, 20mM to 0.5M, 30mM to 0.5M, 50 mM to 0.5M, 0.1M to 0.5M, 0.2M to 0.5M, 50nM to 0.2M, 100nM to 0.2M, 200nM to 0.2M, 500nM to 0.2M, 1mM to 0.2M, 10mM to 0.2 M, 20mM to 0.2M, 30mM to 0.2M, 50mM to 0.2M, 0.1M to 0.2M, 50nM to 0.1M, 100nM to 0.1M, 200nM to 0.1M, 500nM to 0.1M, 1mM to 0.1M, 10mM to 0.1M, 20mM to 0.1M, 30mM to 0.1M, 50mM to 0.1M, 50nM to 50mM, 100nM to 50mM, 200nM to 50mM, 500nM to 50mM, 1mM to 50mM, 10mM to 50mM, 20mM to 50mM, 30mM to 50mM, 50nM to 30mM, 100nM to 30mM, 200nM to 30mM, 500nM to 30mM, 1mM to 30mM, 10mM to 30mM, 20mM to 30 mM, 50nM to 20mM, 100nM to 20mM, 200nM to 20mM, 500nM to 20mM, 1mM to 20mM, 10mM to 20mM, 50nM to 10mM, 100nM to 10mM, 200nM to 10mM, 500nM to 10 mM, 1mM to 10mM, 50nM to 1mM, 100nM to 1mM, 200nM to 1mM, 500nM to 1mM, 50nM to 500nM, 100nM to 500nM, 200nM to 500nM, or 100nM to 200nM.

In some embodiments, the buffer solution has a pH of 2.0 to 2.5, 2.0 to 3.0, 2.0 to 3.5, 2.0 to 4.0, 2.0 to 4.5, 2.0 to 5.0, 2.5 to 3.0, 2.5 to 3.5, 2.5 to 4.0, 2.5 to 4.5, 2.5 to 5.0, 3.0 to 3.5, 3.0 to 4.0, 3.0 to 4.5, 3.0 to 5.0, 3.5 to 4.0, 3.5 to 4.5, 3.5 to 5.0, 4.0 to 4.5, 4.0 to 5.0, or 4.5 to 5.0.

In some embodiments, the cosolvent has a concentration of 1 to 2% (v/v), 1 to 3% (v/v), 1 to 4% (v/v), 1 to 5% (v/v), 1 to 6% (v/v), 1 to 7% (v/v), 1 to 8% (v/v), 1 to 9% (v/v), 1 to 10% (v/v), 2 to 3% (v/v), 2 to 4% (v/v), 2 to 5% (v/v), 2 to 6% (v/v), 2 to 7% (v/v ), 2 to 8% (v/v), 2 to 9% (v/v), 2 to 10% (v/v), 3 to 4% (v/v), 3 to 5% (v/v), 3 to 6% (v/v), 3 to 7% (v/v), 3 to 8% (v/v), 3 to 9% (v/v), 3 to 10% (v/v ), 4 to 5% (v/v), 4 to 6% (v/v), 4 to 7% (v/v), 4 to 8% (v/v), 4 to 9% (v/v ), 4 to 10% (v/v), 5 to 6% (v/v), 5 to 7% (v/v), 5 to 8% (v/v), 5 to 9% (v/v), 5 to 10% (v/v), 6 to 7% (v/v), 6 to 8% (v/v), 6 to 9% (v/v), 6 to 10% (v/v), 7 to 8% (v/v), 7 to 9% (v/v), 7 to 10% (v/v), 8 to 9% (v/v), 8 to 10% (v/v), or 9 to 10% (v/v).

In another aspect, the present invention provides a decontamination preparation, which comprises the decontamination composition according to any one of the items of the first aspect.

In some embodiments, the hydrogen peroxide and other ingredients of the decontamination composition are located in the same formulation unit.

In some embodiments, the hydrogen peroxide and other ingredients of the decontamination composition are located in different formulation units.

In some embodiments, the decontamination preparation is a decontamination solution.

In another aspect, the present invention provides a protective equipment, which comprises the decontamination composition according to any one of the items of the first aspect or the decontamination preparation according to any one of the items of the second aspect.

In another aspect, the present invention provides a use of the decontamination composition according to any one of the items of the first aspect, the decontamination preparation according to any one of the items of the second aspect, or the protective equipment according to any one of the items of the third aspect in the decontamination of a chemical poison.

In some embodiments, the chemical poison is a nerve agent or a blister agent.

In some embodiments, the nerve agent is an organophosphorus agent, for example selected from the group consisting of sarin, tabun, soman, and VX.

In some embodiments, the blister agent is mustard gas, Lewisite, nitrogen mustard, or a combination thereof, such as a combination of Lewisite and mustard gas.

In another aspect, the present invention provides a method for decontaminating a chemical poison, which comprises the following steps:
(1) rapidly mixing ingredients of the decontamination composition according to any one of the items of the first aspect, or the decontamination preparation according to any one of the items of the second aspect, to obtain a mixture system;
(2) pouring or spraying or contacting the mixture system to an area, such as water, soil or biological surface, where the chemical poison leaks.

In some embodiments, the chemical poison is a nerve agent or a blister agent.

In some embodiments, the nerve agent is an organophosphorus agent, for example, selected from the group consisting of sarin, tabun, soman, and VX.

In some embodiments, the blister agent is mustard gas, Lewisite, nitrogen mustard, or a combination thereof, such as a combination of Lewisite and mustard gas.

### Beneficial effects of the present invention

The decontamination formulation of the present invention aims at mustard gas, Lewisite or Agent Yellow, and the decontamination product is non-toxic and non-irritating to living organisms. Thorough decontamination is achieved within 1 minute. The enzyme consumption is only at nanomolar level. It is efficient, safe and environmentally friendly, thereby minimizing the subsequent impact of leakage accidents.

### Brief Description of the Drawings

The drawings described herein are used to provide a further understanding of the present invention and constitute a part of the present application. The illustrative examples of the present invention and their descriptions are used to explain the present invention and do not constitute an improper limitation of the present invention. In the attached drawings:

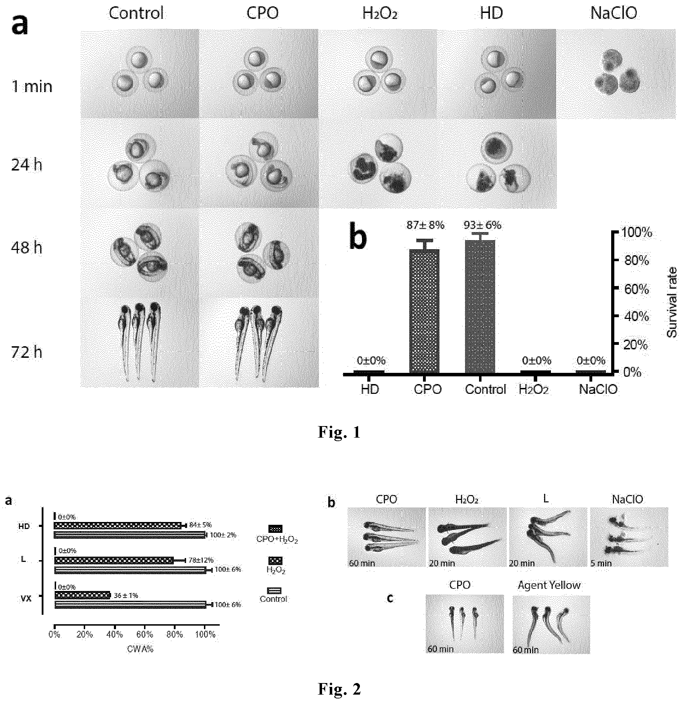

Fig. 1: (a) the photos under light microscope, which show the comparison of effect on the survival of zebrafish embryos, of the group using the decontamination formulation with the group using only hydrogen peroxide, of the control group of mustard gas without being decontaminated, and of the groups using traditional decontaminants; (b) survival rate of embryos.
Fig. 2: (a) the decontamination effect on the chemical poisons of mustard gas, Lewisite and VX, of the group using the decontamination formulation, of the group using only hydrogen peroxide, and of the chemical poison control group; (b) in the case of Lewisite, the response of zebrafish larvae to the decontamination formulation; (c) in the case of Agent Yellow, the response of zebrafish larvae to the decontamination formulation (the group of the decontamination formulation of the present invention, and the chemical poison control group).

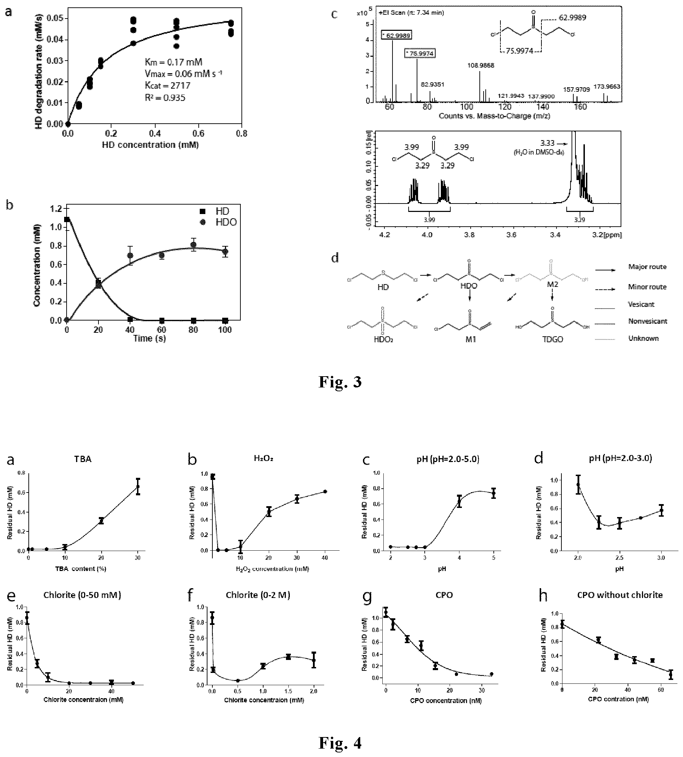

Fig. 3: (a) Michaelis-Menten analysis of enzymatic oxidation of mustard gas; (b) kinetics of mustard gas degradation and mustard sulfoxide formation; (c) mass spectrum and NMR spectrum of enzymatic degradation products of mustard gas; (d) proposed degradation mechanism of mustard gas.
Fig. 4: the optimal condition exploration diagram of each formula of the decontamination formulation of the present invention.

### Specific Models for Carrying Out the present invention

The technical solutions in the examples of the present invention will be clearly and completely described below with reference to the accompanying drawings in the examples of the present invention. Obviously, the described examples are only some of the examples of the present invention, rather than all the examples. The following description of at least one exemplary example is merely illustrative in nature and is in no way intended to limit the present invention, its application or uses. Based on the examples of the present invention, all other examples obtained by those of ordinary skill in the art without making creative efforts fall within the protection scope of the present invention.

Chloroperoxidase (CPO, EC 1.11.1.10) is a multifunctional glycoprotein secreted by the marine fungus *Caldariomyces fumago,* which is one of the most versatile catalysts in heme enzymes. However, the broad substrate selectivity of CPO results in its poor decomposition efficiency for HD.

Zebrafish (*Danio rerio*) embryos are often used as vertebrate models for environmental and animal physiological monitoring because the models have advantages in terms of chemical permeability, short disease incubation period, sensitivity to chemical treatments, and ease of observation and operation. In addition, the operating conditions of CPO do not conflict with the developmental conditions of zebrafish, which allowed us to establish a new environmental model combining the enzyme and zebrafish embryos to detect decontamination efficiency.

### Example 1:

All procedures were performed in a well-ventilated fume hood at 25±5°C, freshly prepared H₂O₂ stock solution was used in each experiment. Zebrafish embryos were collected from a spawning apparatus, rinsed with embryo medium E3, and divided in a 24-well plate with 10 embryos per well. A pipette was used to carefully remove the culture medium from the wells of the culture plate, then 1ml of test solution was added to the culture well within 4 hours after fertilization, the culture plate was placed flatwise and incubated at 27±1°C, and the embryonic death rate were recorded every day, lasting for 3 days. The data were subsequently expressed as mean ± standard deviation of the results of 3 independent parallel experiments. CPO reaction conditions were optimized by using blank embryo culture medium E3 as negative control, and specific solutions of 0.1-5% (vol%) TBA, 1-100 mM KH₂PO₄, 0.1-50 mMKCl, and 0.05-100 µM HD (pH 3.0-7.0) were added to the embryo culture medium. By establishing a system for degrading 0.1 mM HD in 5 min with 20 nM CPO and 0.2 mM H₂O₂ at pH 4.5, the toxicity of HD and its degradation products was evaluated.

As shown in Fig. 1, all the embryos treated with 0.1 mM HD died within 24h, while the survival rates of the CPO-treated embryos and the negative control embryos were 87± 8% and 93±6%, respectively. The results were expressed as mean ± standard deviation, n=3. In the last two cases, the embryos and larvae showed no malformation or morphological change. However, the embryos exposed to 0.5% sodium hypochlorite were immediately damaged.

### Example 2:

The HD stock solution (0.1 mM), which was obtained by dissolving pure HD in TBA, was added to the buffer solution comprising 0.1 M KH₂PO₄, 0.5 M KCl, 0.022 µM CPO and 5mM H₂O₂ with pH of 2.75 to obtain HD stock solution having a final concentration of 1mM. In another experiment, H₂O₂ was reacted with HD in the absence of CPO to confirm the key role of the enzyme in this process. The control samples were prepared separately by decomposing HD in buffers without CPO or H₂O₂. After reacting for 1 minute, the organic layer was quenched with an equal volume of CH₂Cl₂ and separated, then dried over anhydrous Na₂SO₄, subsequently transferred to a gas chromatography vial and stored at 20°C, and finally analyzed by gas chromatography-mass spectrometry. Similar methods were used to study the degradation of 1mM L and VX under the conditions of CPO concentration of 0.220 µM and reaction time of 1 min and 5 min respectively. It could be seen from Fig. 2a that complete decomposition of HD and LVX was achieved quickly by CPO-catalyzed oxidation with the consumption of enzyme at nanomolar level and H₂O₂ concentration of 5mM, revealing CPO is capable of promoting the degradation of various CWAs simultaneously. When the concentration of H₂O₂ was 1mM, HD and L were completely consumed within 1min, 1mM HD was completely consumed within 40s, and 1mM VX was completely consumed within 5min. Under the conditions that the concentration of H₂O₂ was 5mM and CPO was absent, the residual amounts of HD, L, and VX obtained were 83.85±5.01%, 78.36±11.56%, and 35.72±0.77% after 1h, 1min, and 5min of reaction, respectively. Therefore, except for that the degrading rate of VX reached 60% within 5 minutes, the non-catalytic reaction between the chemical poisons and H₂O₂ were relatively slow.

### Example 3:

Zebrafish larvae three days after fertilization were used to evaluate the degradation efficiency for L and Agent Yellow (HD+L) by CPO. Zebrafish larvae were divided and added into 24-well plates at 6 larvae per well. 0.1 mM L solution, CPO and H₂O₂ were added to the modified embryonic E3 medium, with the final concentration of 20 nM for L and 0.2 mM for CPO and H₂O₂ respectively, and the reaction lasted for 5 minutes. Additional experiments were performed with samples comprising only 0.1 mM L and 0.1 mM L + 0.2 mM H₂O₂ in embryonic E3 medium. In addition, 0.5% sodium hypochlorite was tested as an oxidizing agent. The degradation of a mixture of 0.1 mM HD and 0.1 mM L was studied in 20 nM CPO and 0.5 mM H₂O₂, respectively. In the modified embryonic E3 medium comprising TBA (0.1%) and KH₂PO₄ (1-10mM), the optimal reaction conditions for HD were determined by adjusting pH to 4.5 with HCl. In the CPO system, L could be effectively degraded, while the oxidation speed without CPO was not fast enough and toxic light absorption products could be produced, as shown in Figs 2b to 2c. In the presence of 0.5% sodium hypochlorite, zebrafish larvae were immediately damaged, died within a few minutes, and decomposed after 5 minutes. In 1mM L, cessation of heartbeat was observed within 20 minutes, and the body of dead larvae was twisted and had a corrosive appearance. The larvae treated with 0.1 mM L and 0.2 mM H₂O₂ died within 20 minutes and had dark skin. Similar results were observed for the degradation of Agent Yellow at 0.1 mM HD + 0.1 mM L. That was to say, CPO could effectively degrade HD and L, so it could be used as a real chemical weapons decontamination agent for the degradation of Agent Yellow.

### Example 4:

The optimal conditions were set as: 0.1 M KH₂PO₄, 0.5 M KCl, pH 2.75, 5% TBA, 5mM H₂O₂, 20nM CPO, temperature of 25±2°C, and total volume of 7ml. The degradation rate of HD catalyzed by CPO was monitored under optimal reaction conditions. Every 5 seconds or 10 seconds, 1 ml of the reaction mixture was taken out and immediately mixed with 1 ml of CH₂Cl₂ under stirring with a magnetic stirrer for 1 minute to obtain a sample for later detection. Michaelis-Menten analysis was used to determine the affinity of CPO for HD, and GraphPad Prism 5 was used for graphing analysis. As shown in Fig. 3, the *K*ₘ, *V*ₘₐₓ, and *k*_{cat} values were 0.17 mM, 0.06 mM s⁻¹ (*R*² = 0.935), 2717M⁻¹s⁻¹, respectively. For extremely-efficient enzymes, the reaction rates exceeded 10⁷ M⁻¹s⁻¹, while for ultra-efficient enzymes, the *k*_{cat}/*K*ₘ ratios were 10⁸ to 10¹⁰ M⁻¹s⁻¹. The corresponding value of CPO was 1.58×10⁷ M⁻¹s⁻¹, indicating that CPO had the ability to effectively promote the HD oxidation.

Various modifications of the present invention in addition to those described herein will become apparent to those skilled in the art from the foregoing description. Such modifications are also intended to fall within the scope of the appended claims. Each reference cited in the present application, including all patents, patent applications, journal articles, books, and any other publications, is hereby incorporated by reference in its entirety.

## Claims

1. A decontamination composition, which comprises a chloroperoxidase;
preferably, the chloroperoxidase is CPO (EC 1.11.1.10).

2. The decontamination composition according to claim 1, which further comprises one or more selected from the group consisting of hydrogen peroxide, halide ion, buffer solution and cosolvent;
preferably, the decontamination composition further comprises an ingredient or combination selected from the group consisting of: hydrogen peroxide; halide ion; buffer solution; cosolvent; hydrogen peroxide and halide ion; hydrogen peroxide and buffer solution; hydrogen peroxide and cosolvent; halide ion and buffer solution; halide ion and cosolvent; buffer solution and cosolvent; hydrogen peroxide, halide ion, and buffer solution; hydrogen peroxide, halide ion, and cosolvent; hydrogen peroxide, buffer solution, and cosolvent; halide ion, buffer solution, and cosolvent; and hydrogen peroxide, halide ion, buffer solution and cosolvent.

3. The decontamination composition according to claim 1 or 2, **characterized by** one or more of the following:
(1) the halide ion is selected from the group consisting of fluoride ion, chloride ion and bromide ion; preferably chloride ion;
(2) the buffer solution is selected from the group consisting of phosphate buffer solution, borate buffer solution and citrate buffer solution; preferably phosphate buffer solution, such as KH₂PO₄ buffer solution;
(3) the cosolvent is selected from alcohols, such as tert-butyl alcohol.

4. The decontamination composition according to any one of claims 1 to 3, **characterized by** one or more of the following:
(1) the chloroperoxidase has a concentration of nanomolar level to millimolar level; for example, 1 nM to 100 mM, preferably 20 nM to 10 mM;
(2) the hydrogen peroxide has a concentration of 0 to 50 mM;
(3) the halide ion has a concentration of 0 to 0.5M;
(4) the buffer solution has a pH of 2.0 to 5.0;
(5) the cosolvent has a concentration of 1 to 10% (v/v).

5. A decontamination preparation, which comprises the decontamination composition according to any one of claims 1 to 4;
preferably, the hydrogen peroxide and other ingredients in the decontamination composition are located in the same preparation unit;
preferably, the hydrogen peroxide and other ingredients in the decontamination composition are located in different preparation units;
preferably, the decontamination preparation is a decontamination solution.

6. A protective equipment, which comprises the decontamination composition according to any one of claims 1 to 4 or the decontamination preparation according to claim 5.

7. Use of the decontamination composition according to any one of claims 1 to 4, the decontamination preparation according to claim 5 or the protective equipment according to claim 6 in the decontamination of a chemical poison;
preferably, the chemical poison is a nerve agent or a blister agent;
preferably, the nerve agent is an organophosphorus agent, for example selected from the group consisting of sarin, tabun, soman and VX;
preferably, the blister agent is mustard gas, Lewisite, nitrogen mustard or a combination thereof, for example, a combination of Lewisite and mustard gas.

8. A method for decontaminating a chemical poison, which comprises the following steps:
(1) rapidly mixing the ingredients of the decontamination composition according to any one of claims 1 to 4, or the decontamination preparation according to claim 5, to obtain a mixture system;
(2) pouring or spraying or contacting the mixture system to an area, such as water, soil or biological surface, where the chemical poison leaks;
preferably, the chemical poison is a nerve agent or a blister agent;
preferably, the nerve agent is an organophosphorus agent, for example, selected from the group consisting of sarin, tabun, soman, and VX;
preferably, the blister agent is mustard gas, Lewisite, nitrogen mustard, or a combination thereof, for example, a combination of Lewisite and mustard gas.
